# EUROPÄISCHE PATENTSCHRIFT

(11) **EP 0 685 580 B1**
(45) Veröffentlichungstag und Bekanntmachung des Hinweises auf die Patenterteilung: **15.03.2000**
(21) Anmeldenummer: 95107638.9
(22) Anmeldetag: 19.05.1995
(51) Int. Cl.: D01H 13/22

(54) **Verfahren und Vorrichtung zur Ermittlung von Ursachen für Fehler in Garnen, Vorgarnen und Bändern**
Method and device for determining causes of faults in yarns, rovings and slivers
Procédé et dispositif pour déterminer les causes de défauts des fils, mèches et rubans de fibres

(30) Priorität: 02.06.1994 CH 172794
(43) Veröffentlichungstag der Anmeldung: 06.12.1995
(73) Patentinhaber: ZELLWEGER LUWA AG, 8610 Uster (CH)
(72) Erfinder: Meier, Rudolf, CH-8192 Glattfelden (CH); Aemmer, Peter F., Dr., CH-8907 Wettswil (CH)
(74) Vertreter: Ellenberger, Maurice

(56) Entgegenhaltungen:
- WO-A-93/05477
- DE-A- 3 928 417

## Beschreibung

Die Erfindung betrifft ein Verfahren und eine Vorrichtung zur Ermittlung von produktionsbedingten Ursachen für Fehler in Garnen, Vorgarnen und Bändern.

Fehler in Produkten wie Garnen, Vorgarnen und Bändern können im Prinzip zwei Ursachen haben. Entweder liegt die Ursache in der Beschaffenheit des Rohmaterials oder sie liegt in der Art und Weise wie das Produkt hergestellt wird, also im Herstellungsprozess. Beide Ursachen können zu Fehlern führen, die ähnlich aussehen. Solche Fehler sind Dick- oder Dünnstellen in einem Garn, die sich mehr oder weniger weit erstrecken oder periodisch auftretende Fehler, sogenannte Moiré-Effekte. Die nachfolgend beschriebene Erfindung bezieht sich ausschliesslich auf Besonderheiten oder Fehler, deren Ursache bei den Produktionsapparaten oder Produktionsverfahren zu suchen ist.

Es sind sehr gute Geräte bekannt, mit denen Fehler in einem Garn erkannt werden können. Es ist üblich, störende Fehler im Produkt zu beheben, indem man sie herausschneidet. Es ist ferner üblich, aus den vorhandenen Fehlern in Garnen Rückschlüsse auf die Verwendung oder Weiterbehandlung des Garnes oder auf die Qualität des Ausgangsmateriales, des Rohstoffes, zu ziehen.

Aus der Internationalen Patentanmeldung WO 93/05477, die den der Erfindung am nächsten kommenden Stand der Technik aufzeigt, ist beispielsweise ein System zur Erstellung einer Störungsdiagnose an Produktionsmaschinen und eine Anwendung des Systems an Textilmaschinen bekannt, bei dem die Störungsdiagnose anhand einer wissensbasierten Auswertung von Signalen erfolgt, die die Qualität der hergestellten Produkte signalisieren. Dazu wird eine sogenannte Wissensbasis verwendet, in der die Maschinenteile einer Produktionsmaschine beschrieben sind zum Zweck später daraus logische Schlussfolgerungen zu ziehen. Dabei sind diese Maschinenteile in eine speziell ausgebildete Hierarchie von Regeln abgebildet. Solche Maschinenteile sind beispielsweise Zahnräder (die durch ihre Zähnezahl beschrieben sind), Hebel (die durch ihre Länge beschrieben sind) usw. Aus an sich bekannten und in Spektrogrammen dargestellten Signalen, die Aussagen über die Qualität der hergestellten textilen Produkte machen, werden charakteristische Abweichungen ermittelt und daraus mögliche Störungsursachen abgeleitet.

Ein Nachteil solcher Verfahren und Vorrichtungen besteht darin, dass damit nur mögliche Ursachen für periodische Fehler angegeben werden können. Ein Beispiel dafür sind die sogenannten Moiré-Effekte in Bändern oder Garnen.

Die Erfindung hat somit zum Ziel, die genannten Mängel zu beheben und ein Verfahren und eine Vorrichtung zu schaffen, die es erlauben, aus Fehlern in den genannten Produkten, deren Ursachen auf besonders einfache Art zu ermitteln, sofern diese Ursachen im Produktionsprozess liegen.

Dieses Ziel wird durch das erfindungsgemäße Verfahren des Anspruchs 1 bzw. durch dieVorrichtung des Anspruchs 7 erreicht.

Die Erfindung besteht somit darin dass in einem ersten Schritt die Fehler im Produkt mit einem sensor erfasst, da heisst erkannt werden. Erkannt werden sie, indem gemessene Werte des Produktes einen oder mehrere Schwellwerte überschreiten. Für die Erkennung ist in einer vorzugsweisen Ausführung ein Parameter-Paar, z.B. bei einem Garn der Querschnitt und die Länge eines Fehlers massgebend. Die Werte für diese zwei Parameter können in einem rechtwinkligen Koordinatensystem mit Fehlerlänge und Fehlerquerschnitt als Abszisse bzw. Ordinate eingetragen werden. Dieses so gebildete "Klassierfeld" kann in einen Raster aus Längen- bzw. Querschnittsklassen eingeteilt sein. Die ermittelten Werte der erkannten Fehler werden automatisch laufend den vorgegebenen Klassen zugeordnet, so dass sich daraus ein Fehlermuster oder eine Charakteristik ergibt. Dieses Fehlermuster wird mit vorgegebenen modellhaften Bezugsmustern verglichen, die auf Ursachen für die erfassten Fehler schliessen lassen. Die vorgegebenen Bezugsmuster können durch vorausgehende Untersuchungen ermittelt oder aus der Erfahrung stammen.

Die Erfassung der Fehler geschieht in an sich bekannter Art für Garn beispielsweise in einer Garnreinigeranlage, die vorzugsweise "on-line" arbeitet und mehrere Spinnstellen, beispielsweise einer Rotorspinnmaschine, überwacht. Ein Beispiel eines Klassiersystems für Fehler ist aus der Schweizer Patentschrift Nr. 477 573 bekannt. Bezugsgrössen oder Schwellwerte, die Grenzwerte für die Identifizierung und Klassifizierung von Fehlern angeben, werden empirisch aufgrund von speziellen Anwenderbedürfnissen ermittelt. Bei der laufenden Prüfung des Garnes werden in an sich bekannter Weise die Fehler automatisch den einzelnen Klassen zugeordnet und für die einzelnen Klassen gezählt. Das dabei entstandene Fehlermuster kann graphisch dargestellt und auch graphisch interpretiert werden, um auf eine Fehler-Ursache zu schliessen. Ebenso kann das Fehlermuster auch in einem digitalen Speicher abgespeichert sein, von dem ein Rechner die Werte beziehen und in entsprechender Weise zueinander in Beziehung setzen kann, wie das eben durch die Interpretation durch den Menschen anhand von graphisch dargestellten Mustern auch erfolgt.

Gemäss einer besonderen Ausführung, die insbesondere bei Rotorspinnmaschinen angewandt werden kann, wo in bekannter Weise alle Spinnstellen durch Garnreiniger überwacht werden, sollen alle Garnfehler aus allen Spinnstellen in der obengenannten Weise klassiert werden. Daraus kann ein Durchschnittsfehlermuster abgeleitet werden, das für jede Klasse durchschnittliche Fehlerzahlen angibt. Parallel dazu soll für jede Spinnstelle ein Fehlermuster erstellt werden. Dann werden die Fehlermuster jeder einzelnen Spinnstelle mit dem Durchschnittsfehlermuster verglichen. Erzeugt eine Spinnstelle ein abweichendes Fehlermuster, so wird dieses im Hinblick auf eine Aussage über eine mögliche Fehlerursache untersucht, indem man es mit vorbekannten Fehlermustern für bekannte Fehlerursachen vergleicht.

Das erfindungsgemässe Verfahren und die erfindungsgemässe Vorrichtung haben bedeutende Vorteile. Fehlerursachen können damit sowohl in einem Labor, durch Untersuchung von Stichproben oder direkt im Produktionsprozess durch Untersuchung der ganzen Produktionsmenge ermittelt werden. Die Fehlerquelle kann ohne störenden Eingriff in die Produktion des Produktes gesucht werden, was bedeutet, dass damit auch kleine, sonst unbeachtete Fehlerursachen erkannt werden können oder, dass das allmähliche Entstehen von solchen Fehlerursachen ab einem frühen Stadium erfasst und verfolgt werden kann. Als Bezugsgrössen genügen relativ wenige Referenzwerte. Somit kann der Vergleich zwischen den ermittelten Werten aus der Produktion und den Bezugsgrössen durch den Menschen oder durch einen Rechner erfolgen, wofür schon einfache Rechner genügen. Es ist auch möglich, einen Produktionsapparat also eine Spinnmaschine in relativ wenige Teile aufzuteilen und damit Bezugswerte für Schäden an diesen wenigen Teilen zu speichern und dabei schon eine einfache aber brauchbare Diagnose zu erhalten. Zudem erlaubt es die Erfindung auch nichtperiodische Fehler in einem Produkt zu erfassen und deren Ursachen zu finden.

Im folgenden wird die Erfindung anhand eines Beispiels und mit Bezug auf die beiliegenden Zeichnungen näher erläutert, wobei
Figur 1 in schematischer Darstellung eine erfindungsgemässe Vorrichtung bei der das Produkt "on-line" erfasst wird,
Figur 2 in schematischer Darstellung eine erfindungsgemässe Vorrichtung bei der Proben der Produkte "off-line" erfasst werden,
Figur 3, 4, 5, 6 und 7 je eine andere Charakteristik,
Figur 8 eine weitere schematische Darstellung einer Charakteristik,
Figur 9 ein Prinzipschema der Erfindung und
Figur 10 Häufigkeitsverteilungen von Zählwerten und von Bezugswerten zeigt.

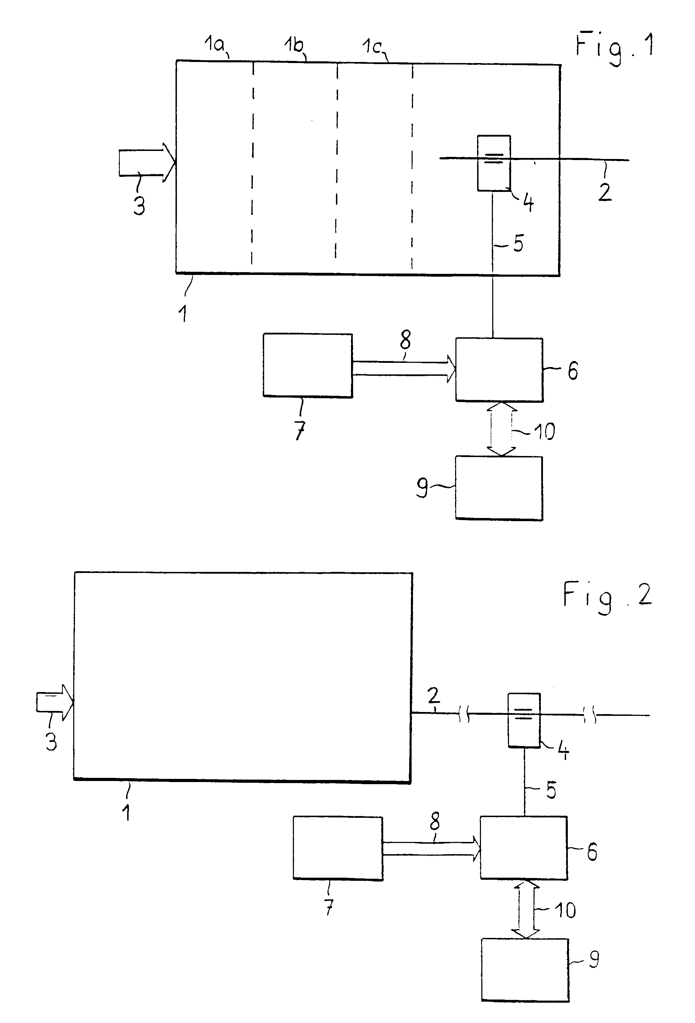

Figur 1 zeigt eine Produktionsvorrichtung 1 für ein Produkt wie ein Garn, Vorgarn oder ein Band. Die Produktionsvorrichtung 1 kann demnach beispielsweise als Spinnmaschine, Flyer, Karde, Strecke, Kämmaschine usw. ausgebildet sein, in der ausgehend von bekannten Vorprodukten 3, wie z. B. Baumwollfasern usw. Garn, oder aus einem Band ein Garn hergestellt wird. Die Produktionsvorrichtung 1 weist einen an sich bekannten Sensor 4 auf, der hier beispielsweise als Mittel zum Erfassen von Fehlern in einem Garn dient. Damit können bestimmte Eigenschaften des Garnes 2 erfasst werden. Der Sensor 4 gibt über eine Leitung 5 elektrische Signale an eine an sich bekannte Auswerteeinheit 6 ab. Sensor 4 und Auswerteeinheit 6 sind beispielsweise Teil eines bekannten Garnreinigers und bilden somit sowohl einen Teil eines Mittels zum Erfassen der Fehler wie auch eines Mittels zum Klassieren der Fehler. Als Mittel zum Klassieren der Fehler in einem Garn ist der USTER CLASSIMAT bestens bekannt. Damit werden bestimmte Fehler erfasst. Welche Fehler genau erfasst werden, ist durch die fest eingestellten Grenzwerte für Parameter bestimmt, durch die die Fehler charakterisiert sind. Die erfassten Fehler, beispielsweise die Verdickungen im Garn, werden zur Länge, also zu Menge des gesponnenen Garnes in Bezug gesetzt und wahlweise auch gezählt, indem die Auswerteeinheit gleich klassierte Fehler zählt und die Zählwerte für jede Klasse speichert. Mit 9 ist eine Recheneinheit bezeichnet, die über einen Bus 10 mit der Auswerteeinheit 6 verbunden ist und die beispielsweise auch einen Speicher umfasst, in dem Bezugsgrössen oder Kombinationen von Bezugsgrössen gespeichert sind, die beispielsweise zusammengenommen eine Charakteristik oder ein Bezugsmuster ergeben. Die Auswerteeinheit 6 und die Recheneinheit 9 bilden zusammen ein Mittel zum Zählen und Klassieren der Fehler, sowie ein Mittel zum Ableiten und Erstellen eines Fehlermusters oder Bezugsmusters. Dafür ist die Recheneinheit 9 mit einem Programm versehen, das gemäss den nachfolgend gemachten Angaben erstellt werden kann. Die Dateneingabe- und Ausgabeeinheit 7 kann auch Mittel zur Bilddarstellung wie einen Bildschirm umfassen. Es ist auch so zu verstehen, dass die Recheneinheit 9 einen Speicher umfasst, in dem vorgegebene Bezugsmuster gespeichert werden können.

Figur 2 zeigt eine Vorrichtung wie sie im Prinzip schon aus der Figur 1 hervorgeht, aber mit dem Unterschied, dass der Sensor 4 ausserhalb der Produktionsmaschine 1 und nicht notwendigerweise im Produktionsstrang angeordnet ist. Gleiche Elemente sind deshalb mit gleichen Bezugszeichen wie in Figur 1 versehen. Die Anordnung gemäss Figur 2 eignet sich deshalb auch zur Durchführung eines Produktionsprozesses, bei dem das Produkt aus dem Prozess entnommen und in einem gesonderten Labor untersucht wird. Dies kann insbesondere durch die Entnahne von Stichproben geschehen.

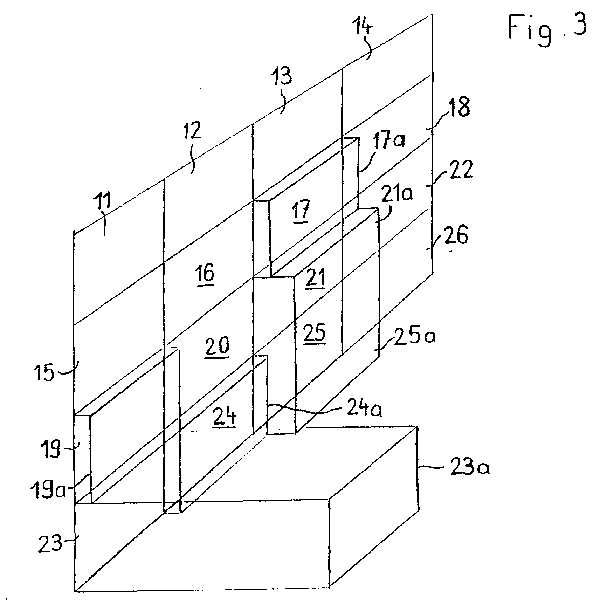

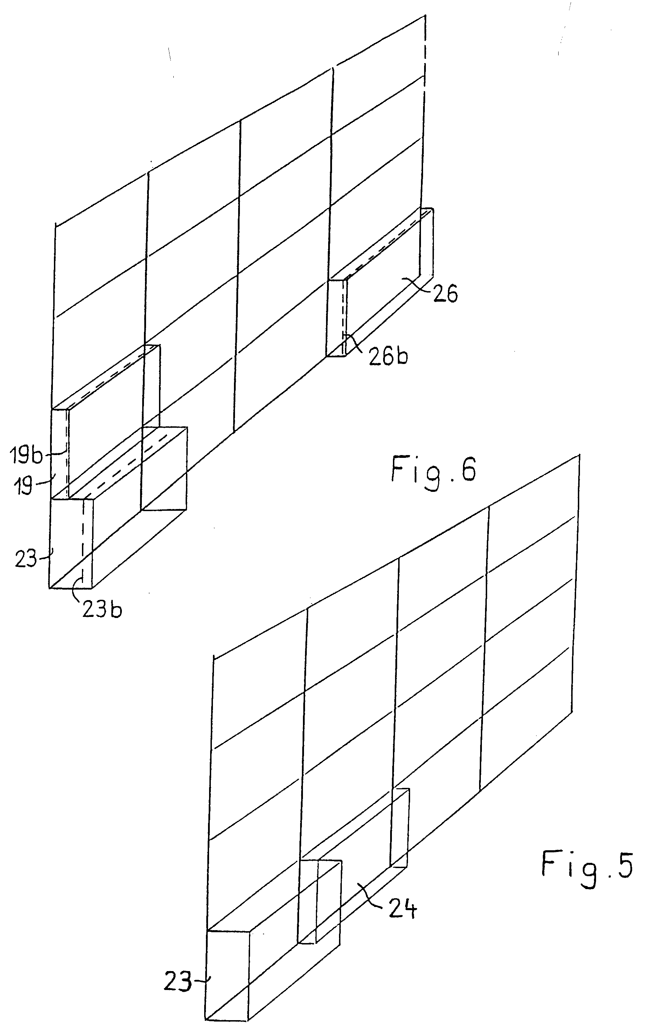

Figur 3 zeigt in vereinfachter Darstellung eine sogenannte und bestens bekannte USTER CLASSIMAT - Tabelle wie sie in der obengenannten Schweizer Patentschrift Nr. 477 573 (Fig. 5) ausführlicher dargestellt ist. Eine Vereinfachung besteht hier darin, dass nur sechzehn Felder dargestellt sind, die mit Bezugszeichen 11 bis 26 versehen sind. Jedes dieser sechzehn Felder definiert für ein Garn einen Fehlertyp, der durch eine bestimmte Länge und eine bestimmte Abweichung vom mittleren Querschnitt charakterisiert ist. Jedes der genannten Felder entspricht einer Klasse von möglichen Garnfehlern und alle Felder zusammen ergeben das Klassierfeld. Über den mit 17, 19, 21, 23, 24 und 25 bezeichneten Feldern sind Balken 17a, 19a, 21a, 23a, 24a und 25a aufgetragen, deren Höhe oder Länge je einer Anzahl gemessener Fehler in der betreffenden Klasse entspricht. Die Gesamtheit der dargestellten Balken 17a bis 25a sowie die weiteren hier nicht besonders hervorgehobenen Balken der Höhe Null, wie beispielsweise über den Feldern 11 bis 16, ergeben zusammen eine Charakteristik oder ein Fehlermuster, das sich aus Zählwerten für bestimmte Fehler zusammensetzt. In dieser Form ergibt sich eine dreidimensionale Bilddarstellung zur Darstellung von drei Parametern. Diese sind hier für ein Garn, die Länge eines Fehlers, der Querschnitt oder die Masse eines Fehlers und die Anzahl der Fehler pro Klasse.

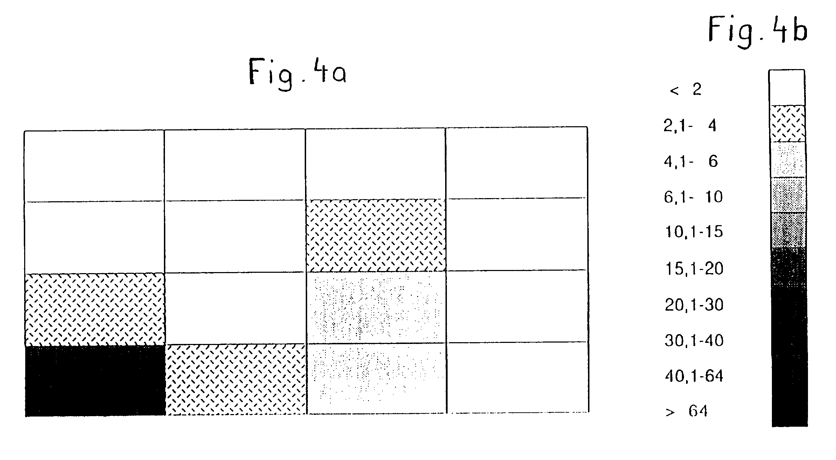

Figur 4a zeigt eine Klasseneinteilung wie sie aus der Figur 3 bereits bekannt ist, bei der aber die Zählwerte statt durch Balken durch Muster oder Farben dargestellt sind. Figur 4b zeigt eine dazugehörende Tabelle, die angibt welche Zählwerte welchen Mustern oder Farben zuzuordnen sind. Dies für das gleiche Fehlermuster, das bereits in der Figur 3 dargestellt ist.

Figur 5 zeigt eine entsprechende Darstellung wie sie schon aus der Figur 3 bekannt ist, die aber andere Fehler oder Zählwerte angibt. Hier sind durch zwei Balken lediglich Zählwerte für die Felder 23 und 24 angegeben. Diese beiden Zählwerte ergeben beispielsweise eine Charakteristik für ein fehlerfreies Garn oder, genauer für ein Garn das zwar auch mit den gesetzten Grenzwerten erkennbare Fehler aufweist, die aber als unerheblich beurteilt werden. Diese Charakteristik ist als normal anzusehen. Charakteristiken wie sie die vorgehend beschriebenen Figuren 3 und 4 zeigen, weichen erheblich von dieser ab und deuten damit auf Fehler hin, die nicht annehmbar sind.

Figur 6 zeigt Zählwerte für die Felder oder Klassen 19, 23 und 26 in der bekannten Darstellung. Diese weisen wiederum auf eine andere Ursache für die Fehler hin.

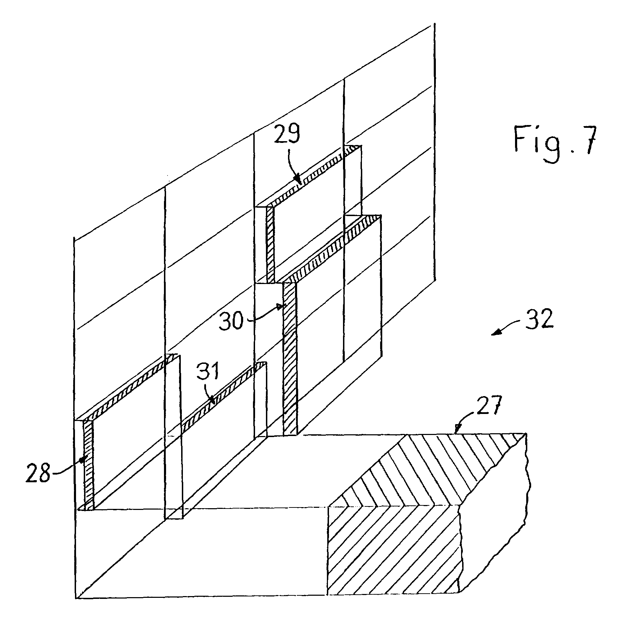

Figur 7 zeigt eine Darstellung entsprechend derjenigen von Figur 3 bei der Bereiche 27, 28, 29, 30 und 31 angegeben sind, die für eine bestimmte Ursache für Fehler anzeigen, in welchen Bereichen die Zählwerte der einzelnen Klassen liegen müssen, damit auf diese bestimmte erwartete Ursache geschlossen werden kann.

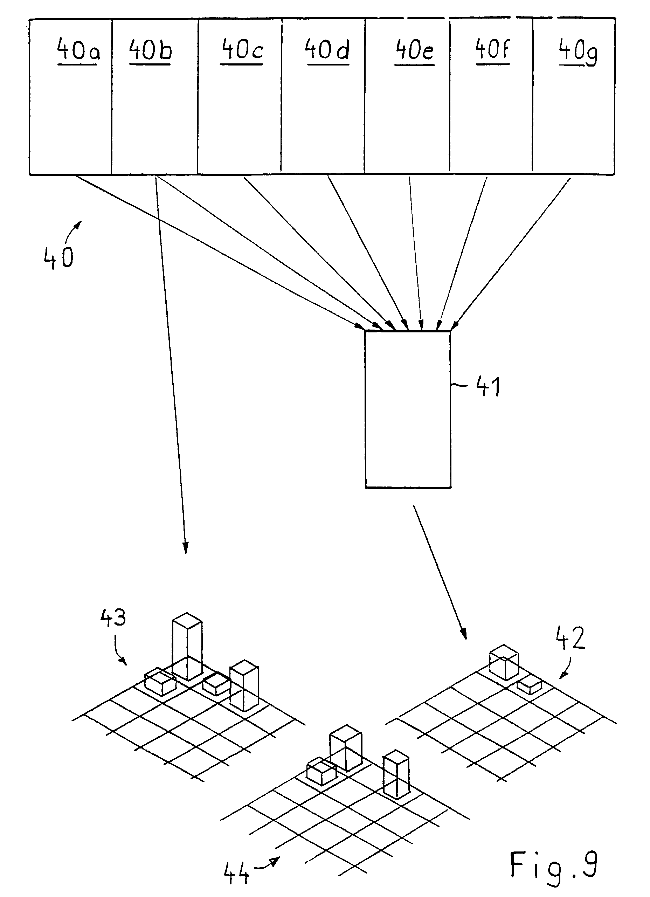

Ein Vergleich der Figuren 3, 5 und 6 miteinander zeigt drei verschiedene Charakteristiken oder Fehlermuster mit Erhebungen und Senken. In Figur 4 ist das dreidimensionale Fehlermuster aus Figur 3 in ein zweidimensionales Fehlermuster oder Bild umgesetzt. Durch praktische Versuche an der Produktionsvorrichtung 1 kann ermittelt werden, welche Charakteristik oder welches Fehlermuster der vorgenannten Art durch bestimmte Veränderungen an einzelnen Teilen oder Baugruppen der Produktionsvorrichtung erhalten wird. Beispielsweise haben praktische Versuche an einer Rotorspinnmaschine gezeigt, dass ein Fehlermuster wie es die Figuren 3, 4 und 7 zeigen dann entsteht, wenn der Rotor fehlerhaft ist. Die Figur 6 dagegen zeigt ein Fehlermuster, das typischerweise entsteht, wenn eine Abzugswalze fehlerhaft ist. Die Figur 5 dagegen zeigt ein Fehlermuster, das von einem Garn stammt, das als fehlerfrei gilt, oder das eben nur solche Fehler enthält, die auf keinen Fehler im Produktionsprozess hindeuten. Die hier gezeigte Charakteristik bestehend aus den Zählwerten die den Feldern zugeordnet sind, bildet eine Bezugsgrösse für die Charakteristiken, die in den Figuren 3, 6 und 7 gezeigt sind. Da in einer Spinnmaschine immer mehrere Spinnstellen gleichzeitig parallel zueinander arbeiten, können auch die anderen Spinnstellen die gewünschten Bezugsgrössen abgeben oder es können sogar Mittelwerte aus Warten aller Spinnstellen gebildet werden, um davon die Bezugsgrössen für fehlerfreies Garn abzuleiten. Dies geht beispielsweise aus der Figur 9 hervor.

Figur 9 zeigt in schematischer Darstellung eine Produktionsmaschine 40, die hier beispielsweise als Spinnmaschine ausgebildet ist, und die eine Vielzahl parallel arbeitender und unter sich gleicher Elemente, hier die Spinnstellen 40a bis 40g aufweist, die mit gleichartigem Material gespiesen werden. Jede Spinnstelle liefert in an sich bekannter Weise ein Garn mit möglichst gleichen Eigenschaften wie es beispielsweise von einer Durchschnittsspinnstelle 41 geliefert werden könnte, die hier stellvertretend für die Summe der realen Spinnstellen 40a usw. dargestellt ist. Die Durchschnittsspinnstelle 41 liefert Garn, das bei der Fehlerprüfung ein Fehlermuster ergibt, wie es mit 42 bezeichnet ist. Das Fehlermuster 42 zeigt zulässige Fehlerzahlen in den Klassen an und entspricht etwa demjenigen von Fig. 5. Zu diesem durchschnittlichen Fehlermuster hat auch eine Spinnstelle 40b beigetragen, die Garn mit mehr Fehlern produziert, wie dies mit dem Fehlermuster 43 angedeutet werden soll. Der Unterschied oder die Differenz zwischen den beiden Fehlermustern 43 und 42 ergibt ein weiteres Fehlermuster 44. Für die Identifikation einer Fehlerursache in der Spinnstelle 40b kann man nun diekt vom Fehlermuster 43 oder vom weiteren Fehlermuster 44 ausgehen und eines dieser Fehlermuster 43, 44 mit einem vorgegebenen Bezugsmuster vergleichen. Das Bezugsmuster ist diesen Fehlermustern sehr ähnlich, im Idealfall sogar identisch. Es zeigt also in denselben Feldern eine vergleichbare oder in den Fehlermustern 43, 44 überschrittene Anzahl Fehler an.

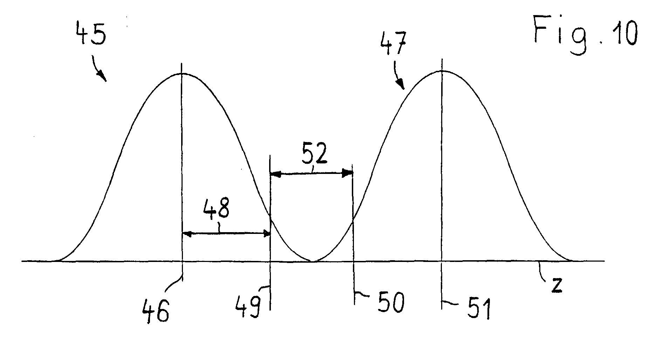

Figur 10 zeigt eine Darstellung von Häufigkeitsverteilungen, wie sie für Zählwerte von Fehlern auftreten können. Mit Z ist eine Achse bezeichnet, auf der Zählwerte aufgetragen sind. Senkrecht darüber sind für solche Zählwerte die Zahlen aufgetragen, die angeben wie oft solche Zählwerte gezählt wurden. Eine Verteilung 45, welche um einen Zählwert 46 gelegt ist, gibt somit an, dass der Zählwert 46 der häufigste Zählwert ist, dass daneben aber auch noch weitere grössere und kleinere Zählwerte gezählt werden konnten. Die Verteilung 45 gehört zum Klassierfeld eines Bezugsmusters, während mit 47 eine Verteilung bezeichnet ist, die zum gleichen Klassierfeld des Fehlermusters gehört. In einem Abstand 48 vom Zählwert in Richtung zunehmender Werte ist eine Grenze angegeben, die nach bestimmten und wählbaren Kriterien ermittelt ist und die nachfolgend als obere Vertraunsgrenze 49 bezeichnet wird. In gleicher Weise ist für die Zählwerte der Verteilung 47 des Fehlermusters eine untere Vertrauensgrenze 50 zum Zählwert 51 angegeben. Wichtig ist dabei der Umstand, dass dafür gesorgt ist, dass die beiden Vertrauensgrenzen in einem positiven Abstand 52 zueinander stehen. Diese Vertrauensgrenzen sind dann wichtig, wenn es darum geht, zu entscheiden ob in einer Klasse eine relevante Anzahl Fehler vorliegt, die aussergewöhnlich ist.

Für das erfindungsgemässe Verfahren wird demnach zuerst ein Produktionsapparat gedanklich in Teile 1a, 1b, 1c usw. aufgeteilt, in denen man Fehler, Abnützungserscheinungen, Verschmutzungen oder andere Effekte aufspüren möchte, die zu Qualitätseinbussen im Produkt 2 führen. Anschliessend wird durch eine Versuchsreihe ermittelt, welche charakteristischen Fehlerbilder oder Fehlermuster sich ergeben, wenn einer der betreffenden Teile 1a, 1b, 1c, usw. unerwünschte Veränderungen erfahren hat. Dazu werden über den Sensor 4 die Fehler im Produkt 2 erfasst und in der Auswerteeinheit 6 mit Grenzwerten oder mit Wertebereichen verglichen, die der Klassierung entsprechen. Für jede Klasse der Klassierung, d.h. für jedes Feld 11 bis 26 aus Figur 3, 4, 5, 6 oder 7 werden nun diejenigen Fehler am produzierten Produkt gezählt, die gemäss der Klassierung als Fehler gelten. Diese Zählwerte sind statistisch ermittelte Werte. Für sie gelten die Gesetzmässigkeiten der Statistik und damit können sie streuen und die Streuung kann in einer Verteilungskurve, z.B. der Poisson'schen oder vereinfacht der Gauss'schen-Verteilungskurve dargestellt werden. Werden viele Versuche an der Produktionsmaschine gemacht und damit viele Werte gewonnen, die sich auf eine grosse Menge produzierten Gutes beziehen, so ergeben sich Zählwerte, die eine geringe Streuung aufweisen und die Verteilungskurve ist entsprechend schmal. Aus diesen Zählwerten, die für eine bestimmte und beispielsweise künstlich herbeigeführte Fehlerursache gelten, können Bezugsmuster oder Charakteristiken 32 (Fig. 7) abgeleitet werden, die aus einer Kombination von Zählwerten für die Felder eines Klassierfeldes gelten. Solche Bezugsmuster sind in den Figuren 3, 4, 6, 7 und 8 dargestellt. Auch das Fehlermuster 44 aus Fig. 9 gehört dazu.

Während der Produktion des Produktes 2, beispielsweise eines Garnes, laufen teilweise die gleichen Vorgänge ab. Das Produkt 2 wird durch den Sensor 4 laufend überwacht. Treten Fehler auf, so werden diese Fehler vorgegebenen Klassen zugeordnet und in den einzelnen Klassen gezählt. So werden für jedes Feld 11 bis 26 laufend Zählwerte ermittelt. Die den verschiedenen Klasssen zugeordneten Zählwerte ergeben zusammen ein Fehlermuster. Die Fehlermuster werden durch Normierung oder Hochrechnung auf eine bestimmte Garnlänge, üblicherweise 100km, bezogen. Anschliessend werden typische und wichtige Hinweise enthaltende Fehlermuster aus der Menge der vorliegenden Fehlermuster durch eine Ähnlichkeitsbetrachtung mit den oben beschriebenen Bezugsmustern ermittelt. Zu jedem Bezugsmuster gehört ein bestimmter Hinweis auf einen Fehler im Produktionsprozess und damit ist eine Fehlerursache ermittelt.

Es gibt aber auch Fehlermuster, die zwar durch die laufende Überwachung des Produktes 2 ermittelt werden, die aber zulässig sind oder eben nicht auf Fehler im Produktionsprozess hinweisen. Solche Fehlermuster sind in der Figur 5 und in der Figur 9 (42) gezeigt. Solche Fehlermuster treten immer auf und es ist notwendig, diese in der Auswertung zu berücksichtigen. Dazu gibt es zwei Möglichkeiten.

Eine erste Möglichkeit besteht darin, Zählwerte solcher Fehlermuster von den Zählwerten der übrigen Fehlermuster abzuziehen, wie das für die Fig. 9 beschrieben ist. Dieses Vorgehen ist dann gerechtfertigt, wenn solche unerheblichen Fehler auch schon beim Erstellen des Bezugsmusters von den erheblichen Fehlern abgezogen wurden. Die so erhaltenen Fehlermuster betreffen dann immer erhebliche Fehler.

Eine zweite Möglichkeit besteht darin, davon auszugehen, dass solche Fehlermuster, die nur unerhebliche Fehler enthalten einfach nicht weiter zu beachten und davon auszugehen, dass solche immer auch in allen Fehlermustern vorhanden sind. Dann erhalten wir Fehlermuster mit unerheblichen Fehlern, für die es keine Bezugsmuster gibt und Fehlermuster mit erheblichen Fehlern, für die es Bezugsmuster gibt. Nur diese letzteren Fehlermuster werden dann herausgesucht.

Da wir hier statistisch ermittelte Werte mit weiteren ebenfalls statistisch ermittelten Werten vergleichen, können Unsicherheiten entstehen, die zu Fehlinterpretationen führen könnten. Diese sollen durch die nachfolgend beschriebenen Verfahrensschritte vermieden werden.

Zu den aus der Versuchsreihe ermittelten Zählwerten 46 (Fig. 10) aus jeder Klasse wird eine obere Vertrauensgrenze 49 ermittelt. Diese liegt oberhalb dem eigentlichen Zählwert 46 und wird nun als eigentlicher Schwellwert für diese Klasse betrachtet. Das Bezugsprofil, dessen Grundlage statistisch ermittelte Werte sind, ist nun durch diese Schwellwerte 49 gebildet.

Zu den aus der Produktion oder aus einzelnen Produktionsstellen ermittelten Zählwerten 51 wird nun die untere Vertrauensgrenze 50 ermittelt. Diese liegt in einem mehr oder weniger grossen Abstand (in Zählwerten gemessen) unterhalb dem eigentlichen Zählwert. Ein Verfahren dazu ist beispielsweise aus der EP-A-03659-01 bekannt.

Für eine Klasse ergibt sich nur dann ein signifikannter Wert für das Fehlermuster, wenn der Zählwert aus der Produktion mit seiner unteren Vertrauensgrenze 50 oberhalb der oberen Vertrauensgrenze 49 des entsprechenden Wertes aus der Versuchsreihe liegt. Oder, mit anderen Worten, wenn ein positiver Abstand 52 zwischen den Vertrauensgrenzen auftritt, so liegt eine Alarmsituation in einer Klasse vor. Um auf eine konkrete Fehlerursache schliessen zu können muss dies möglicherweise für mehrere Klassen gelten, die so ein Fehlermuster ergeben. Das Fehlermuster muss nun mit dem Bezugsmuster verglichen werden, was durch die obengenannte Ähnlichkeitsbetrachtung geschieht.

Im Sinne des oben beschriebenen Verfahrens können die Grenzwerte, wie sie in Figur 6 beispielsweise nit Linien 19b, 23b und 26b dargestellt sind, als untere Vertrauensgrenzen im Sinne der Fig. 10 aufgefasst werden. An diese unteren Vertrauensgrenzen anschliessend können die genannten Zählwerte auch mit einer Toleranzbreite ergänzt werden. So entstehen Bereiche 27 bis 31 wie sie in Figur 7 dargestellt sind und damit auch ein charkteristisches Fehlermuster, das in der Einheit 7 angezeigt oder auch im Rechner 9 gespeichert werden kann.

Alle mit dem obengenannten Verfahren verbundenen Auswertungen werden in der Recheneinheit 9 durchgeführt, die im Programmspeicher entsprechende Programme enthält und die die gemessenen Werte aus der Auswerteeinheit 6 bzw. deren Datenspeicher entnimmt.

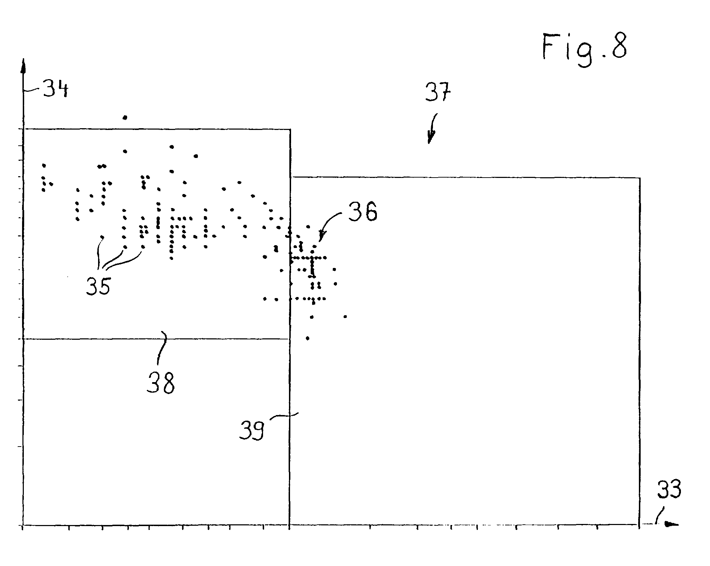

Figur 8 zeigt als weiteres Beispiel eine Klassierung mit unendlich feiner Auflösung, d.h. die einzelnen Felder, die eine Klasse bilden sind unendlich klein, oder, mit anderen Worten, es gibt hier unendlich viele Klassen. Auf einer Achse 33 seien beispielsweise Werte für die Länge der Fehler aufgetragen, während auf einer Achse 34 die Zunahmen des Querschnitts eines Garnes, die eben den Fehler ausmachen, aufgetragen sind. Die einzelnen Punkte 35 geben somit einen gemessenen Fehler mit seiner Länge und seinem Querschnitt an. Da aus den einzelnen Punkten 35 nicht hervorgeht, ob ein oder mehrere Fehler mit diesen Parametern erkannt wurden, werden die Fehler nicht gezählt. Trotzdem ergibt sich so ein Fehlerbild oder eine Charakteristik deren Besonderheit darin liegt, dass an einer Stelle 36 eine Häufung von Ereignissen, also erkannten Fehlern, auftritt, die wiederum einen Hinweis auf eine Ursache für einen Fehler an der Spinnmaschine gibt. Wie hier gezeigt, ist es auch möglich, das Klassierfeld 37, das durch die beiden Achsen 33 und 34 aufgespannt wird, in Bereiche 38 und 39 zu unterteilen, so dass beispielsweise die relative Lage von Häufungen 36 klarer erkannt werden kann. Solche Bereiche können auch für eine differenzierte Wertung oder Gewichtung der einzelnen Ereignisse benützt werden. Um einen Hinweis auf eine Fehlerursache zu erhalten, muss auch in diesem Falle eine Ähnlichkeitsbetrachtung des Fehlermusters wie es durch die Häufung 36 gegeben ist, mit einem gegebenen Bezugsmuster erfolgen.

Eine solche Ähnlichkeitsbetrachtung zwischen einem Fehlermuster und einem Bezugsmuster kann entweder mit nur binären Angaben oder auch mit diskreten Werten durchgeführt werden. Auf jeden Fall ist durch die vorgehend beschriebenen Verfahrensschritte das Problem textiler Natur auf ein Problem der sogenannten Symbolerkennung zurückgeführt worden. Danach kann jedes Fehlermuster und jedes Bezugsmuster als Symbol aufgefasst werden. Das Fehlermuster soll nun mit dem Bezugsmuster durch an sich bekannte Mittel der Symbolerkennung verglichen, d.h. auf Übereinstimmung geprüft werden. Dazu gibt es viele bekannte Verfahren.

Für binär vorliegende Fehler- und Bezugsmuster, wie beispielsweise jenes aus Fig. 8, ist beispielsweise ein Verfahren wie es R. Lippmann in "An Introduction to Computing with Neural Nets" aus IEEE, Acoustics, Speech and Signal Processing Magazine, April 1987, Seiten 4 - 22, beschreibt geeignet. Solche Verfahren sind beispielsweise für Apparate, die Schrifterkennung durchführen, bekannt und realisiert.

Für Fehler- und Bezugsmuster, die mit diskreten Zählwerten vorliegen, sind viele Verfahren zur Mustererkennung anwendbar. Eines davon ist im Buch von T. Tilli: "Mustererkennung mit Fuzzy Logic", Franzis Verlag, 1993, beschrieben. Natürlich können auch eigene Algorithmen entwickelt werden, die die gestellte Aufgabe erfüllen und die genaue Regeln über die zulässigen und die unzulässigen Abweichungen zwischen Fehlermustern und Bezugsmustern angeben.

Es ist weiter zu bemerken, dass nicht nur Dickstellen in einem Garn Fehler bilden. Auch Dünnstellen sind als Fehler anzusehen, die eine Aussage über Fehlerquellen an der Spinnmaschine erlauben. Neben den gezeigten Klassierfeldern für zwei oder drei Parameter sind auch Klassierfelder denkbar, die mehr als drei Parameter darstellen. Ein solcher weiterer Parameter, der auch seine Bedeutung in diesem Zusammenhang hat, ist die Länge des fehlerfreien Garns zwischen einzelnen Fehlern oder der Abstand zwischen den einzelnen Fehlern. Dabei kann man bezüglich Fehlerfreiheit noch unterscheiden, zwischen Abständen zwischen gleichartigen Fehlern und Abständen zwischen verschiedenartigen Fehlern. Je grösser die Anzahl solcher Parameter, desto differenzierter ist die Aussage über die Ursache von Fehlern. Damit steigt aber auch der Aufwand zur Analyse an. Es ist ebenso möglich, mit nur einem Parameter zu arbeiten. Das würde in der Darstellung gemäss Fig. 3 z.B. bedeuten, dass das Feld nur in Spalten oder Zeilen unterteilt wird und dass sich die Zählwerte der Balken 17a, 21a und 25a über die ganze Spalte verteilen würden, wenn eine Aufteilung nach Spalten erfolgt.

Das erfindungsgemässe Verfahren kann auch in einer nachfolgenden Produktionsstufe durchgeführt werden, um Fehler in der vorausgehenden Produktionsstufe zu finden. Beispielsweise kann dies für Garne in der Spulerei durchgeführt werden um damit Ursachen für Fehler in den vorgelagerten Ringspinnstufen aufzufinden.

In der Praxis können Fehler, die durch den verwendeten Rohstoff verursacht werden, und Fehler, die durch den Produktionsprozess verursacht werden gemeinsam auftreten. Diese können mit Hilfe der vorliegenden Erfindung auseinandergehalten werden, denn die Maschinenfehler erzeugen immer charakteristische Fehlermuster. Diese können durch die Rohstofffehler zwar verzerrt sein, die Maschinenfehler ergeben aber immer erkennbare Fehlermuster. Bei parallel laufenden Produktionsprozessen kann es deswegen vorteilhaft sein Fehlermuster, die auf unzulässige Fehler hinweisen, in einem ersten Auswahlschritt auszusondern, indem man diese mit einem durchschnittlichen Fehlermuster vergleicht, das zulässige Fehler über alle Produktionsprozesse gemittelt anzeigt. Nur davon abweichende Fehlermuster müssen dann noch mit vorgegebenen Bezugsmuster verglichen werden.

## Patentansprüche

1. Verfahren zur Bereitstellung von Daten für die Ermittlung von produktionsbedingten Ursachen für Fehler in Garnen, Vorgarnen und Bändern (2), dadurch gekennzeichnet, dass die Fehler über mindestens einen Parameter mit einem Sensor (4) erfasst werden, dass Parameter zur automatischen Zuweisung der Fehler zu Klassen verwendet werden und dass aus den erfassten und den Klassen zugewiesenen Fehlern ein Fehlermuster (32) abgeleitet wird, das einen Bezug zur Ursache für den produktionsbedingten Fehler darstellt.

2. Verfahren nach Anspruch 1, dadurch gekennzeichnet, dass das Fehlermuster eine Menge von gezählten und den Klassen zugeordneten Fehlern über mehrere Klassen (23, 24) ist.

3. Verfahren nach Anspruch 1, dadurch gekennzeichnet, dass bei parallel zueinander arbeitenden gleichartigen Produktionsvorrichtungen (40a, 40b, 40c), die mit gleichartigem Material gespiesen werden, je ein Fehlermuster (32, 43) für jede Produktionsvorrichtung abgeleitet wird, dass jedes Fehlermuster mit einem gemeinsamen Bezugsmuster verglichen wird und dass aus diesem Vergleich eine Fehlerursache bestimmt wird.

4. Verfahren nach Anspruch 3, dadurch gekennzeichnet, dass
aus den Fehlermustern jeder Produktionsvorrichtung ein mittleres Fehlermuster (42) erstellt wird, dass
jedes einzelne Fehlermuster mit dem mittleren Fehlermuster verglichen wird, dass
in jedem Fehlermuster nach einer signifikanten Abweichung zum mittleren Fehlermuster gesucht wird und dass
aus dem eine signifikante Abweichung enthaltenden Fehlermuster (44) die Ursache für die Fehler ermittelt wird.

5. Verfahren nach Anspruch 1 oder 3, dadurch gekenrzeichnet, dass der Bezug zur Ursache mit Hilfe eines Verfahrens zur Mustererkennung hergestellt wird.

6. Verfahren nach Anspruch 3, dadurch gekennzeichnet, dass für jede Klasse Vorgaben (19b, 23b, 26b) gemacht werden, die die Zählwerte in Bezug zur Menge des untersuchten Garns setzen.

7. Vorrichtung zur Bereitstellung von Daten für die Ermittlung von produktionsbedingten Ursachen von Fehlern in Garnen, Vorgarnen und Bändern, gekennzeichnet durch einen Sensor (4) zum Erfassen des Umfangs einzelner Fehler, durch ein Mittel (7, 8) zum automatischen Klassieren der erfassten Fehler und durch ein Mittel (8, 9) zum Ableiten und Erstellen eines Fehlermusters aus den gezählten und klassierten Fehlern.

8. Vorrichtung nach Anspruch 7, gekennzeichnet durch ein Mittel (6, 9) zum Zählen der erfassten Fehler.

9. Vorrichtung nach Anspruch 7, ferner gekennzeichnet durch ein Mittel zum Speichern (9) vorgegebener Fehlermuster und zum Vergleichen (6) eines vorgegebenen Bezugsmusters mit einem aktuell ermittelten Fehlermuster.

10. Vorrichtung nach Anspruch 7, dass als Mittel (6, 7, 8, 9, 10) zum Ableiten und Erstellen eines Fehlermusters aus den gezählten und klassierten Fehlern eine Einrichtung zur Mustererkennung vorgesehen ist.

## Claims

1. A method for preparing data for determining production-related causes of faults in yarns, rovings and slivers (2), characterised in that the faults are detected via at least one parameter using a sensor (4), parameters are used for automatically allocating the faults to classes and from the determined faults allocated to classes a fault pattern (32) is derived, which represents a reference for the cause of the production-related fault.

2. A method according to claim 1, characterised in that the fault pattern is a quantity of faults which have been counted and allocated to classes over a plurality of classes (23, 24).

3. A method according to claim 1, characterised in that, with like production devices (40a, 40b, 40c) operating parallel to one another and supplied with like material, a fault pattern (32, 43) is derived for each production device, each fault pattern is compared with a common reference pattern and a fault cause is determined from this comparison.

4. A method according to claim 3, characterised in that a mean fault pattern (42) is produced from the fault patterns of each production device, each individual fault pattern is compared with the mean fault pattern, a search is carried out in each fault pattern for a significant deviation from the mean fault pattern and the cause for the fault is determined from the fault pattern (44) containing a significant deviation.

5. A method according to claim 1 or 3, characterised in that the reference for the cause is determined with the aid of a method for pattern recognition.

6. A method according to claim 3, characterised in that, for each class, preset values (19b, 23b, 26b) are determined, which set the counting values with reference to the quantity of the examined yarn.

7. A device for preparing data for determining production-related causes of faults in yarns, rovings and slivers, characterised by a sensor (4) for determining the scope of individual faults, by means (7, 8) for automatically classifying the determined faults and means (8, 9) for deriving and establishing a fault pattern from the counted and classified faults.

8. A device according to claim 7, characterised by means (6, 9) for counting the determined faults.

9. A device according to claim 7, further characterised by means for storing (9) predetermined fault patterns and for comparing (6) a predetermined fault pattern with a currently determined fault pattern.

10. A device according to claim 7, characterised in that a device for pattern recognition is provided as means (6, 7, 8, 9, 10) for deriving and establishing a fault pattern from the counted and classified faults.

## Revendications

1. Procédé pour la mise à disposition de donnée pour déterminer les causes dues à la production de défauts dans des fils, mèches de préparation et rubans (2), caractérisé en ce que les défauts sont détectés selon au moins un paramètre avec un capteur (4), que les paramètres sont utilisés pour l'attribution automatique des défauts à des classes et en ce qu'il est déduit des défauts détectés et attribués aux classes un dessin défectueux (32) qui établit un rapport à la cause du défaut dû à la production.

2. Procédé selon la revendication 1, caractérisé en ce que le dessin défectueux représente une quantité de défauts comptés et attribués aux classes, sur plusieurs classes (23, 24).

3. Procédé selon la revendication 1, caractérisé en ce qu'on obtient dans des dispositifs de production de même type travaillant parallèlement les uns aux autres (40a, 40b, 40c), qui sont alimentés avec le même matériau, respectivement un modèle de défaut (32, 43) pour chaque dispositif de production, en ce que chaque modèle de défaut est comparé avec un modèle de référence commun et en ce qu'il est déterminé à partir de cette comparaison une cause de défaut.

4. Procédé selon la revendication 3, caractérisé en ce qu'il est établi à partir des modèles de défaut de chaque dispositif de production un modèle de défaut moyen (42), en ce que chaque modèle de défaut individuel est comparé avec le modèle de défaut moyen, que dans chaque modèle de défaut, un écart significatif relativement au modèle de défaut moyen est recherché et en ce que, à partir du modèle de défaut (44) présentant un écart significative il est déterminé la cause du défaut.

5. Procédé selon la revendication 1 ou 3, caractérisé en ce que le rapport à la cause est établi à l'aide d'un procédé de détection de modèle.

6. Procédé selon la revendication 3, caractérisé en ce que pour chaque classe on donne des consignes (19b, 23b, 26b) qui mettent les valeurs comptées en rapport avec la quantité du fil examiné.

7. Dispositif pour la mise à disposition de données pour déterminer des causes dues à la production de défauts dama des fils, mèches de préparation et rubans, caractérisé par un capteur (4) pour détecter l'étendue de défauts individuels, par un moyen (7, 8) pour classer automatiquement les défauts détectés et par un moyen (8, 9) pour en déduire et établir un modèle de défauts à partir des défauts comptés et classés.

8. Dispositif selon la revendication 7, caractérisé par un moyen (6, 9) pour compter les défauts détectée.

9. Dispositif selon la revendication 7, caractérisé en outre par un moyen pour le stockage (9) de modèles de défauts prédéterminés et pour la comparaison (6) d'un modèle de référence prédéterminé avec un modèle de defauts déterminé actuellement.

10. Dispositif selon la revendication 7, caractérisé en ce qu'il est prévu une installation pour la détection des modèles, comme moyen (6, 7, 8, 9, 10) pour la dérivation et l'établissement d'un modèle de défauts à partir des défauts comptés et classifiés.
